# EUROPEAN PATENT SPECIFICATION

(11) **EP 0 990 039 B1**
(45) Date of publication and mention of the grant of the patent: **14.09.2005**
(21) Application number: 98935708.2
(22) Date of filing: 15.07.1998
(51) Int. Cl.: C12N 15/67, C12N 15/61

(54) **INCREASING PRODUCTION OF PROTEINS IN GRAM-POSITIVE MICROORGANISMS**
ERHÖHUNG DES PRODUKTIONS VON PROTEINEN IN GRAM-POSITIVE MIKROORGANISMEN
AUGMENTATION DE LA PRODUCTION DE PROTEINES DANS DES MICRO-ORGANISMES GRAM POSITIF

(30) Priority: 16.07.1997 EP 97305288
(43) Date of publication of application: 05.04.2000
(60) Divisional application: 05009420.0
(73) Proprietor: GENENCOR INTERNATIONAL, INC., Palo Alto, California 94304 (US)
(72) Inventor: QUAX, Wilhelmus, J., NL-2253 VB Voorschoten (NL); CALDWELL, Robert, M., Belmont, CA 94002 (US)
(74) Representative: Kiddle, Simon John
(86) International application number: PCT/US1998/014704
(87) International publication number: WO 1999/004019

(56) References cited:
- EP-A- 0 277 563
- EP-A- 0 293 793
- WO-A-93/25676
- US-A- 5 496 719
- ISHIHARA T. ET AL.: "Cloning and characterization of the gene for a protein thiol-disulphide oxidoreductase in Bacillus brevis" JOURNAL OF BACTERIOLOGY, vol. 177, no. 3, February 1977, pages 745-749, XP002088017

## Description

### FIELD OF THE INVENTION

The present invention generally relates to expression of proteins in gram-positive microorganisms and specifically to gram positive microorganism disulfide bond isomerases. The present invention provides expression vectors, methods and systems for the production of proteins in gram-positive microorganisms.

### BACKGROUND OF THE INVENTION

Gram-positive microorganisms, such as members of the group *Bacillus,* have been used for large-scale industrial fermentation due, in part, to their ability to secrete their fermentation products into the culture media. In gram-positive bacteria, secreted proteins are exported across a cell membrane and a cell wall, and then are subsequently released into the external media usually obtaining their native conformation.

The folding of polypeptide chains depends upon the assistance of molecular chaperones and folding catalysts, such as disulfide bond isomerases, which accelerate specific steps in folding. (Hartl et al., 1995, Current Opinion in Structural Biology, 5:92-102). Disulfide bond isomerases can covalently modify proteins by catalyzing specific isomerization steps that may limit the folding rate of some proteins (PCT/US93/09426).

Disulfide bond isomerase catalyzes thiol/disulfide interchange reactions and promotes disulfide formation, isomerization or reduction, thereby facilitating the formation of the correct disulfide pairings, and may have a more general role in the prevention and premature misfolding of newly translocated chains. Disulfide bond isomerase interacts directly with newly synthesized secretory proteins and is required for the folding of nascent polypeptides in the endoplasmic reticulum (ER) of eukaryotic cells.

In spite of advances in understanding portions of the protein secretion machinery in procaryotic cells, the complete mechanism of protein secretion and the mechanisms associated with correct folding of secreted proteins, especially for gram-positive microorganisms has yet to be fully elucidated.

### SUMMARY OF THE INVENTION

The capacity of the secretion machinery of a Gram-positive microorganism may become a limiting factor or bottleneck to the secretion of properly folded proteins or polypeptides and the production of proteins having the correct conformation in secreted form, in particular when the proteins are recombinantly introduced and overexpressed by the host cell. The present invention provides a means for alleviating that bottle neck.

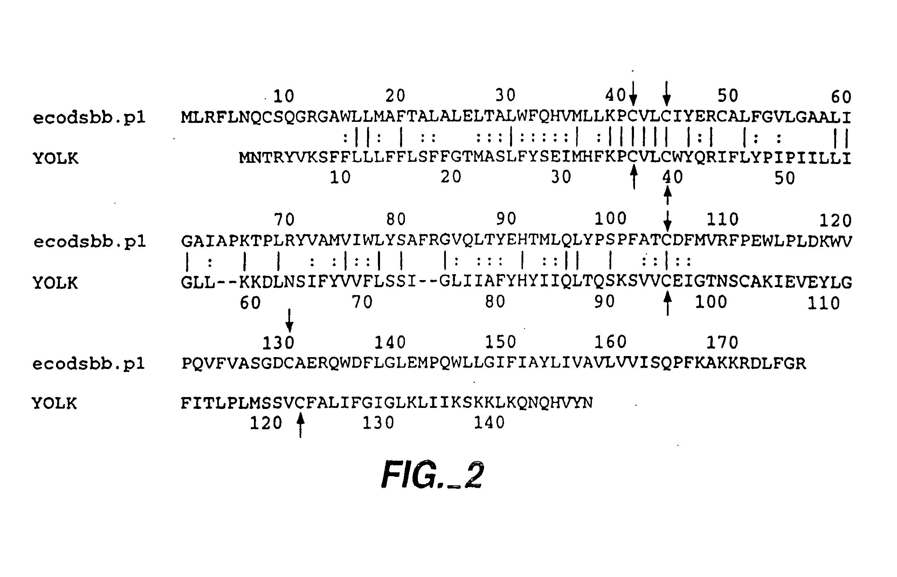

The present invention is based, in part, upon the discovery of a Bacillus subtilis disulfide bond isomerase, Dsb1, identified in heretofore uncharacterised translated genomic DNA by overall amino acid homology with an *E. coli* disulfide bond isomerase. For E.coli disulfide bond isomerase having accession number P30018 and ID number DSBB_ECOLI, it was noted that cysteine residues at 41, 44, 104 and 130 were essential for activity. Those residues are conserved in B.subtilis Dsb1 and noted in Figure 2. The present invention is also based in part on the presence in of potential transmembrane spanning regions in Dsb1.

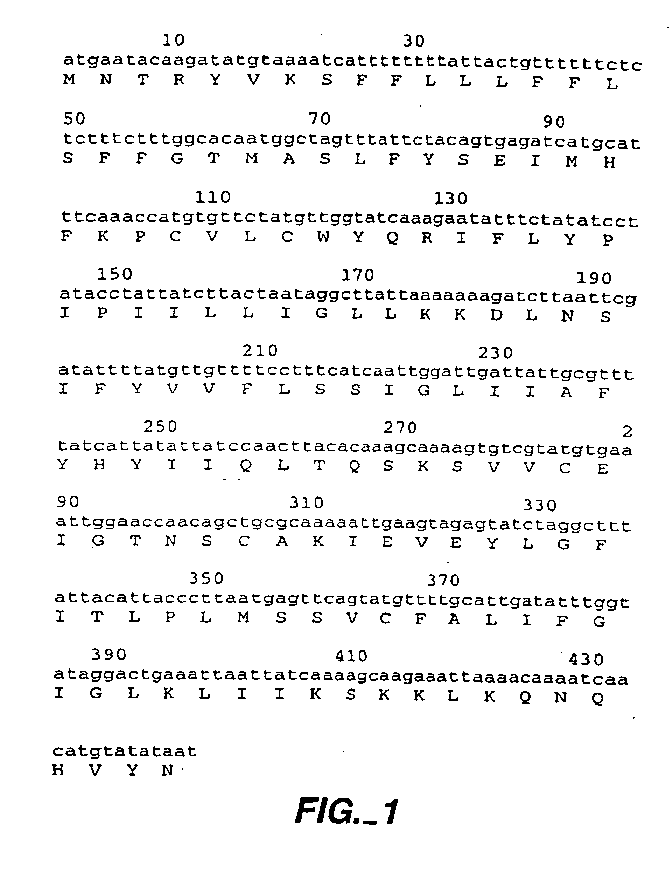

The present invention provides isolated nucleic acid and amino acid sequences for *B. subtilis* Dsb.1 . The amino acid sequence for *B. subtilis* Dsb1 is shown in Figure 1. The nucleic acid sequence encoding *B. subtilis* Dsb1 is shown in Figure 1 .

The present invention also provides improved methods for secreting correctly folded proteins from gram-positive microorganisms. Accordingly, the present invention provides an improved method for secreting a protein in a gram-positive microorganism comprising the steps of obtaining a gram-positive microorganism host cell comprising nucleic acid encoding Dsb1 wherein said nucleic acid is under the control of expression signals capable of expressing said disulfide bond isomerase in a gram-positive microorganism said microorganism further comprising nucleic acid encoding said protein; and culturing said microorganism under conditions suitable for expression of said disulfide bond isomerase and expression and secretion of said protein. In one embodiment of the present invention, the protein is homologous or naturally occurring in the gram-positive microorganism. In another embodiment of the present invention, the protein is heterologous to the gram-positive microorganism.

The present invention provides expression vectors and host cells comprising isolated nucleic acid encoding a gram-positive Dsb1. In one embodiment of the present invention, the host cell comprising nucleic acid encoding Dsb1 is genetically engineered to produce a desired protein, such as an enzyme, growth factor or hormone. In yet another embodiment of the present invention, the enzyme is selected from the group consisting of proteases, carbohydrases including amylases, cellulases, xylanases, reductases and lipases; isomerases such as racemases, epimerases, tautomerases, or mutases; transferases, kinases and phophatases acylases, amidases, esterases, oxidases. In a further embodiment the expression of the disulfide bond isomerase Dsb1 is coordinated with the expression of other components of the secretion machinery. Preferably other components of the secretion machinary, i.e., translocase, SecA, SecY, SecE and/or other secretion factors known to those of skill in the art are modulated in expression at an optimal ratio to Dsb1. For example, it may be desired to overexpress multiple secretion factors in addition to Dsb1 for optimum enhancement of the secretion patterns.

### BRIEF DESCRIPTION OF THE DRAWINGS

Figure 1 shows the nucleic acid (SEQ ID NO:1) and deduced amino acid sequence for Dsb1 (SEQ ID NO:2).
Figure 2 shows an amino acid alignment of *E.coli* disulfide bond isomerase (SEQ ID NO: 3) with the nucleic acid for Dsb1 (YOLK). The cysteine residues equivalent to *E.coli* cysteine residues at 41, 44, 104 and 130 found to be essential for activity of the E.coli disulfide bond isomerase are marked in Figures 2, 3 and 4.

### DETAILED DESCRIPTION

### Definitions

As used herein, the genus *Bacillus* includes all members known to those of skill in the art, including but not limited to *B. subtilis, B. licheniformis, B. lentus, B. brevis, B. stearothermophilus, B. alkalophilus, B. amyloliquefaciens, B. coagulans, B. circulans, B. lautus* and *B. thuringiensis*.

The present invention provides novel Dsb1 disulfide bond isomerases obtainable from gram positive organisms. In a preferred embodiment, the gram-positive organism is *Bacillus*. In another preferred embodiment, the gram-positive organism if from *B. subtilis.* As used herein, the phrase, "*B.subtilis* Dsb1" or "B. subtilis disulfide bond isomerase 1" refers to the amino acid sequence shown in Figure 1. The present invention encompasses amino acid variants of B.subtilis Dsb1 disclosed in Figure 1 that are able to modulate protein folding and/or secretion alone or in combination with other molecular factors, such as secretion factors.

As used herein, "nucleic acid" refers to a nucleotide or polynucleotide sequence, and fragments or portions thereof, and to DNA or RNA of genomic or synthetic origin which may be double-stranded or single-stranded, whether representing the sense or antisense strand. As used herein "amino acid" refers to peptide or protein sequences or portions thereof. A "*B.subtilis* polynucleotide homolog" or "polynucleotide homolog" as used herein refers to a polynucleotide that has at least 80%, at least 90% and at least 95% identity to B. subtilis *Dsb1* or which is capable of hybridizing to B.subtilis Dsb1 under conditions of high stringency and which encodes an amino acid sequence that is able to modulate secretion of the gram-positive microorganism from which it is derived. Modulate as used herein refers to the ability of a disulfide bond isomerase to alter the secretion machinery such that secretion of proteins is altered.

The terms "isolated" or "purified" as used herein refer to a nucleic acid or amino acid that is removed from at least one component with which it is naturally associated.

As used herein, the term "heterologous protein" refers to a protein or polypeptide that does not naturally occur in a gram-positive host cell. Examples of heterologous proteins include enzymes such as hydrolases including proteases, cellulases, amylases, other carbohydrases, reductases and lipases; isomerases such as racemases, epimerases, tautomerases, or mutases, transferases, kinases and phophatases. The heterologous gene may encode therapeutically significant proteins or peptides, such as growth factors, cytokines, ligands, receptors and inhibitors, as well as vaccines and antibodies. The gene may encode commercially important industrial proteins or peptides, such as proteases, carbohydrases such as amylases and glucoamylases, cellulases, oxidases and lipases. The gene of interest may be a naturally occurring gene, a mutated gene or a synthetic gene.

The term "homologous protein" refers to a protein or polypeptide native or naturally occurring in a gram-positive host cell. The invention includes host cells producing the homologous protein via recombinant DNA technology. The present invention encompasses a gram-positive host cell having a deletion or interruption of the nucleic acid encoding the naturally occurring homologous protein, such as a protease, and having nucleic acid encoding the homologous protein, or a variant thereof, re-introduced in a recombinant form. In another embodiment, the host cell produces the homologous protein.

### Detailed Description of the Preferred Embodiments

The present invention provides novel gram-positive microorganism disulfide bond isomerases Dsb1 and methods that can be used in gram-positive microorganisms to ameliorate the bottleneck to secretion of proteins in their native or naturally occurring conformation, in particular when the proteins are recombinantly introduced and overexpressed by the host cell. The present invention provides the disulfide bond isomerase Dsb1 derived from *Bacillus subtilis.*

### I. Dsb1 Nucleic Acid and Amino Acid Sequences

### Nucleic Acid

The *Dsb1* polynucleotide having the sequence as shown in Figure 1 encodes the *Bacillus subtilis* disulfide bond isomerase Dsb1. The *Bacillus subtilis* Dsb1 was identified via a FASTA search of *Bacillus subtilis* uncharacterized translated genomic sequences using an *E.coli* disulfide bond isomerase. The present invention provides gram-positive *Dsb1* polynucleotides which may be used alone or together with factors in a gram-positive microorganism for the purpose of increasing the secretion of desired heterologous or homologous proteins or polypeptides in their native or naturally occurring conformation.

The present invention encompasses novel B.subtilis *Dsb1* polynucleotide homologs encoding gram-positive microorganism Dsb1 which have at least 80%, or at least 90% or at least 95% identity to *B. subtilis* Dsb1 as long as the homolog encodes a protein that is able to function by modulating secretion in a gram-positive microorganism. As will be understood by the skilled artisan, due to the degeneracy of the genetic code, a variety of polynucleotides, i.e.. *Dsb1* polynucleotide variants, can encode the *Bacillus subtilis* disulfide bond isomerases Dsb1. The present invention encompasses all such polynucleotides.

Gram-positive microorganism polynucleotide homologs of *B. subtilis Dsb1* can be identified through nucleic acid hybridization of gram-positive microorganism nucleic acid of either genomic of cDNA origin. The polynucleotide homolog sequence can be detected by DNA-DNA or DNA-RNA hybridization or amplification using B.subtilis *Dsb1* probes, portions or fragments disclosed in the nucleotide sequence shown in the Figures.

Gram-positive microorganism *Dsb1* polynucleotide homologs may be capable of hybridizing to part or all of B.subtilis *Dsb1* under conditions of intermediate to maximal stringency. Hybridization conditions are based on the melting temperature (Tm) of the nucleic acid binding complex, as taught in Berger and Kimmel (1987, Guide to Molecular Cloning Techniques, Methods in Enzymology, Vol 152, Academic Press, San Diego CA), and confer a defined "stringency" as explained below.

"Maximum stringency" typically occurs at about Tm-5°C (5°C below the Tm of the probe); "high stringency" at about 5°C to 10°C below Tm; "intermediate stringency" at about 10°C to 20°C below Tm; and "low stringency" at about 20°C to 25°C below Tm. As will be understood by those of skill in the art, a maximum stringency hybridization can be used to identify or detect identical polynucleotide sequences while an intermediate or low stringency hybridization can be used to identify or detect polynucleotide sequence homologs.

The term "hybridization" as used herein shall include "the process by which a strand of nucleic acid joins with a complementary strand through base pairing" (Coombs J (1994) Dictionary of Biotechnology, Stockton Press, New York NY).
The process of amplification as carried out in polymerase chain reaction (PCR) technologies is described in Dieffenbach CW and GS Dvekster (1995, PCR Primer, a Laboratory Manual, Cold Spring Harbor Press, Plainview NY). A nucleic acid sequence of at least about 10 nucleotides and as many as about 60 nucleotides from the *Dsb1 and Dsb2* nucleotide sequence of Figure 1, preferably about 12 to 30 nucleotides, and more preferably about 20-25 nucleotides can be used as a probe or PCR primer.

### Amino Acid Sequences

The *B. subtilis Dsb1* polynucleotide as shown in Figure 1 encodes *B. subtilis* Dsb1. The present invention encompasses novel gram positive microorganism amino acid variants of the B.subtilis Dsb1 amino acid sequence that are at least 80% identical, at least 90% identical and at least 95% identical to B.subtilis Dsb1 as long as the amino acid sequence variant is able to function in a gram-positive microorganism.

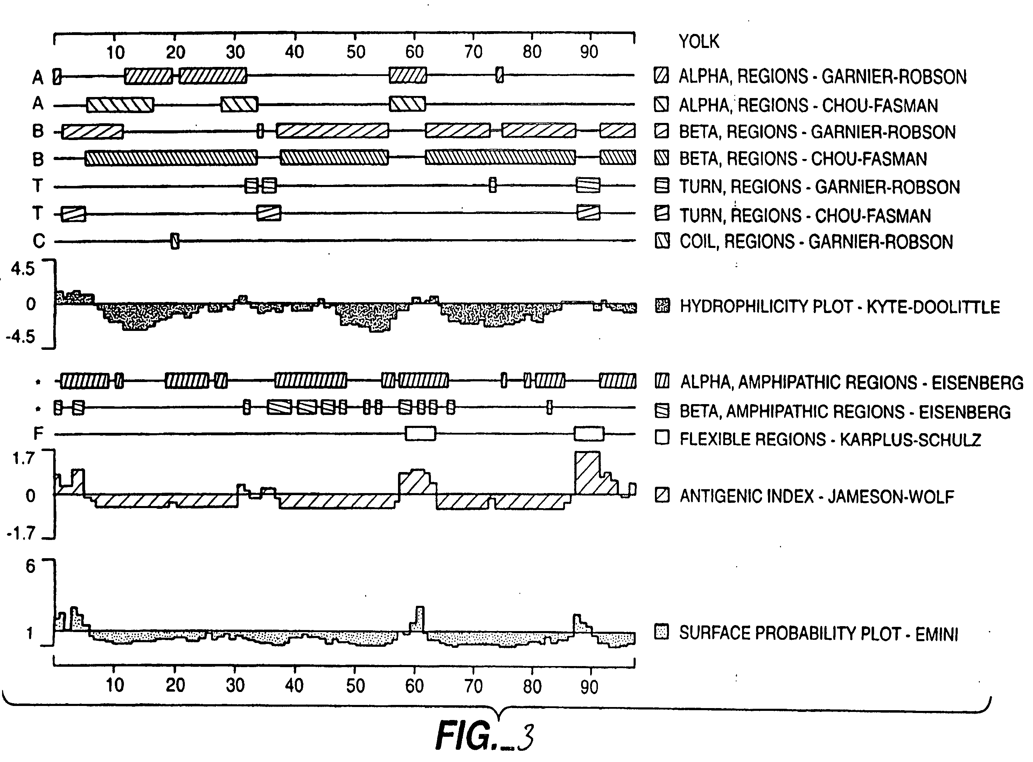

The B. subtilis Dsb1 was discovered upon a FASTA amino acid search of translated B.subtilis genomic DNA sequences vs. An E.coli disulfide bond isomerase amino acid sequence (FASTA search Release 1.0, released on June 11, 1997) parameters being Scoring matrix: GenRunData: blosum50.cmp; variable pamfactor used: Gap creation penalty: 12; and Gap extension penalty: 2). Amino acid alignments are shown in Figures 2 and 3. The hydrophilicity profile for B.subtilis Dsb1, shown in Figure 3, shows potential membrane spanning regions.

### II. Expression Systems

The present invention provides expression systems for the enhanced production and secretion of desired heterologous or homologous proteins in gram-positive microorganisms.

### a. Coding Sequences

In the present invention, the vector comprises at least one copy of nucleic acid encoding a gram-positive microorganism Dsb1 and preferably comprises multiple copies. In a preferred embodiment, the gram-positive microorganism is *Bacillus*. In another preferred embodiment, the gram-positive microorganism is *Bacillus subtilis.* In a preferred embodiment, polynucleotides which encode *B. subtilis* Dsb1, or fragments thereof, or fusion proteins or polynucleotide homolog sequences that encode amino acid variants of Dsb1, may be used to generate recombinant DNA molecules that direct the expression of Dsb1, or amino acid variants thereof, respectively, in gram-positive host cells. In a preferred embodiment, the host cell belongs to the genus *Bacillus.* In another preferred embodiment, the host cell is *B.subtilis.*

As will be understood by those of skill in the art, it may be advantageous to produce polynucleotide sequences possessing non-naturally occurring codons. Codons preferred by a particular gram-positive host cell (Murray E et al (1989) Nuc Acids Res 17:477-508) can be selected, for example, to increase the rate of expression or to produce recombinant RNA transcripts having desirable properties, such as a longer half-life, than transcripts produced from naturally occurring sequence.

Altered gram positive Dsb1 polynucleotide sequences which may be used in accordance with the invention include deletions, insertions or substitutions of different nucleotide residues resulting in a polynucleotide that encodes the same or a functionally equivalent Dsb1 homolog, respectively. As used herein a "deletion" is defined as a change in either nucleotide or amino acid sequence in which one or more nucleotides or amino acid residues, respectively, are absent.

As used herein an "insertion" or "addition" is that change in a nucleotide or amino acid sequence which has resulted in the addition of one or more nucleotides or amino acid residues, respectively, as compared to the naturally occurring gram positive Dsb1.

As used herein "substitution" results from the replacement of one or more nucleotides or amino acids by different nucleotides or amino acids, respectively.

The encoded protein may also show deletions, insertions or substitutions of amino acid residues which produce a silent change and result in a functionally equivalent gram-positive Dsb1 variant. Deliberate amino acid substitutions may be made on the basis of similarity in polarity, charge, solubility, hydrophobicity, hydrophilicity, and/or the amphipathic nature of the residues as long as the variant retains the ability to modulate secretion. For example, negatively charged amino acids include aspartic acid and glutamic acid; positively charged amino acids include lysine and arginine; and amino acids with uncharged polar head groups having similar hydrophilicity values include leucine, isoleucine, valine; glycine, alanine; asparagine, glutamine; serine, threonine, phenylalanine, and tyrosine.

The Dsb1 polynucleotides of the present invention may be engineered in order to modify the cloning, processing and/or expression of the gene product. For example, mutations may be introduced using techniques which are well known in the art, eg, site-directed mutagenesis to insert new restriction sites, to alter glycosylation patterns or to change codon preference, for example.

In one embodiment of the present invention, a Dsb1 polynucleotide may be ligated to a heterologous sequence to encode a fusion protein. A fusion protein may also be engineered to contain a cleavage site located between the Dsb1 nucleotide sequence and the heterologous protein sequence, so that the Dsb1 protein may be cleaved and purified away from the heterologous moiety.

### b. Vector Sequences

Expression vectors used in expressing the disulfide bond isomerases of the present invention in gram-positive microorganisms comprise at least one promoter associated with a gram-positive Dsb1, which promoter is functional in the host cell. In one embodiment of the present invention, the promoter is the wild-type promoter for the selected disulfide bond isomerase and in another embodiment of the present invention, the promoter is heterologous to the disulfide bond isomerase, but still functional in the host cell.

Additional promoters associated with heterologous nucleic acid encoding desired proteins or polypeptides may be introduced via recombinant DNA techniques. In one embodiment of the present invention, the host cell is capable of overexpressing a heterologous protein or polypeptide and nucleic acid encoding one or more disulfide bond isomerase(s) is(are) recombinantly introduced. In one preferred embodiment of the present invention, nucleic acid encoding Dsb1 is stably integrated into the microorganism genome. In another embodiment, the host cell is engineered to overexpress a disulfide bond isomerase of the present invention and nucleic acid encoding the heterologous protein or polypeptide is introduced via recombinant DNA techniques. The present invention encompasses gram-positive host cells that are also capable of overexpressing other disulfide bond isomerases known to those of skill in the art or identified in the future.

In a preferred embodiment, the expression vector contains a multiple cloning site cassette which preferably comprises at least one restriction endonuclease site unique to the vector, to facilitate ease of nucleic acid manipulation. In a preferred embodiment, the vector also comprises one or more selectable markers. As used herein, the term selectable marker refers to a gene capable of expression in the gram-positive host which allows for ease of selection of those hosts containing the vector. Examples of such selectable markers include but are not limited to antibiotics, such as, erythromycin, actinomycin, chloramphenicol and tetracycline.

### c. Transformation

In one embodiment of the present invention, nucleic acid encoding at least one gram-positive disulfide bond isomerase of the present invention is introduced into a gram-positive host cell via an expression vector capable of replicating within the host cell. Suitable replicating plasmids for *Bacillus* are described in Molecular Biological Methods for *Bacillus,* Ed. Harwood and Cutting, John Wiley & Sons. 1990; see chapter 3 on plasmids. Suitable replicating plasmids for *B*. *subtilis* are listed on page 92.

In another embodiment, nucleic acid encoding a gram-positive micro-organism Dsb1 is stably integrated into the microorganism genome. Preferred gram-positive host cells are from the genus *Bacillus.* Another preferred gram-positive host cell is *B*. *subtilis*. Several strategies have been described in the literature for the direct cloning of DNA in *Bacillus.* Plasmid marker rescue transformation involves the uptake of a donor plasmid by competent cells carrying a partially homologous resident plasmid (Contente *et al., Plasmid* 2:555-571 (1979): Haima *et al*., *Mol. Gen. Genet. 223*:185-191 (1990); Weinrauch *et al., J. Bacteriol. 154(3)*:1077-1087 (1983); and Weinrauch *et al., J. Bacteriol. 169(3)*:1205-1211 (1987)). The incoming donor plasmid recombines with the homologous region of the resident "helper" plasmid in a process that mimics chromosomal transformation.

Transformation by protoplast transformation is described for *B. subtilis* in Chang and Cohen, (1979) Mol. Gen. Genet 168:111-115; for B.megaterium in Vorobjeva et al., (1980) FEMS Microbiol. Letters 7:261-263; for B. amyloliquefaciens in Smith et al., (1986) Appl. and Env. Microbiol. 51:634; for B.thuringiensis in Fisher et al., (1981) Arch. Microbiol. 139:213-217; for B.sphaericus in McDonald (1984) J. Gen. Microbiol. 130:203; and B.larvae in Bakhiet et al., (1985) 49:577. Mann et al., (1986, Current Microbiol. 13:131-135) report on transformation of *Bacillus* protoplasts and Holubova, (1985) Folia Microbiol. 30:97) disclose methods for introducing DNA into protoplasts using DNA containing liposomes.

### III. Identification of Transformants

Although the presence/absence of marker gene expression suggests that the gene of interest is also present, its presence and expression should be confirmed. For example, if the nucleic acid encoding Dsb1 is inserted within a marker gene sequence, recombinant cells containing the insert can be identified by the absence of marker gene function. Alternatively, a marker gene can be placed in tandem with nucleic acid encoding the disulfide bond isomerase under the control of a single promoter. Expression of the marker gene in response to induction or selection usually indicates expression of the disulfide bond isomerase as well.

Alternatively, host cells which contain the coding sequence for a disulfide bond isomerase and express the protein may be identified by a variety of procedures known to those of skill in the art. These procedures include, but are not limited to, DNA-DNA or DNA-RNA hybridization and protein bioassay or immunoassay techniques which include membrane-based, solution-based, or chip-based technologies for the detection and/or quantification of the nucleic acid or protein.

The presence of the Dsb1 polynucleotide sequence can be detected by DNA-DNA or DNA-RNA hybridization or amplification using probes, portions or fragments derived from the B.subtilis Dsb1 polynucleotide.

### IV. Secretion Assays

Means for determining the levels of secretion of a heterologous or homologous protein in a gram-positive host cell and detecting secreted proteins include, using either polyclonal or monoclonal antibodies specific for the protein. Examples include enzyme-linked immunosorbent assay (ELISA), radioimmunoassay (RIA) and fluorescent activated cell sorting (FACS). These and other assays are described, among other places, in Hampton R et al (1990, Serological Methods, a Laboratory Manual, APS Press, St Paul MN) and Maddox DE et al (1983, J Exp Med 158:1211).

A wide variety of labels and conjugation techniques are known by those skilled in the art and can be used in various nucleic and amino acid assays. Means for producing labeled hybridization or PCR probes for detecting specific polynucleotide sequences include oligolabeling, nick translation, end-labeling or PCR amplification using a labeled nucleotide. Alternatively, the nucleotide sequence, or any portion of it, may be cloned into a vector for the production of an mRNA probe. Such vectors are known in the art, are commercially available, and may be used to synthesize RNA probes in vitro by addition of an appropriate RNA polymerase such as T7, T3 or SP6 and labeled nucleotides.

A number of companies such as Pharmacia Biotech (Piscataway NJ), Promega (Madison WI), and US Biochemical Corp (Cleveland OH) supply commercial kits and protocols for these procedures. Suitable reporter molecules or labels include those radionuclides, enzymes, fluorescent, chemiluminescent, or chromogenic agents as well as substrates, cofactors, inhibitors, magnetic particles and the like. Patents teaching the use of such labels include US Patents 3,817,837; 3,850,752; 3,939,350; 3,996,345; 4,277,437; 4,275,149 and 4,366,241. Also, recombinant immunoglobulins may be produced as shown in US Patent No. 4,816,567.

### V. Purification of Proteins

Gram positive host cells transformed with polynucleotide sequences encoding heterologous or homologous protein may be cultured under conditions suitable for the expression and recovery of the encoded protein from cell culture. The protein produced by a recombinant gram-positive host cell comprising a disulfide bond isomerase of the present invention will be secreted into the culture media. Other recombinant constructions may join the heterologous or homologous polynucleotide sequences to nucleotide sequence encoding a polypeptide domain which will facilitate purification of soluble proteins (Kroll DJ et al (1993) DNA Cell Biol 12:441-53).

Such purification facilitating domains include, but are not limited to, metal chelating peptides such as histidine-tryptophan modules that allow purification on immobilized metals (Porath J (1992) Protein Expr Purif 3:263-281), protein A domains that allow purification on immobilized immunoglobulin, and the domain utilized in the FLAGS extension/affinity purification system (Immunex Corp, Seattle WA). The inclusion of a cleavable linker sequence such as Factor XA or enterokinase (Invitrogen, San Diego CA) between the purification domain and the heterologous protein can be used to facilitate purification.

### VI. Uses of the Present Invention

### Genetically Engineered Host Cells

The present invention provides genetically engineered host cells comprising nucleic acid encoding gram-positive microorganism Dsb1. In one preferred embodiment, the nucleic acid is stably integrated into the microorganism. In another preferred embodiment, the Dsb1 have the nucleic acid sequence shown in Figure 1. The host cell may comprise nucleic acid encoding additional secretions factors.

The preferred embodiment, the host cell is genetically engineered to produce a desired protein or polypeptide. In a preferred embodiment, the host cell is a *Bacillus.* In another preferred embodiment, the host cell is *Bacillus subtilis.*

In an alternative embodiment, a host cell is genetically engineered to produce a gram-positive Dsb1.

### Polynucleotides

B.subtilis Dsb1 polynucleotides, or any part thereof, provides the basis for detecting the presence of gram-positive microorganism polynucleotide homologs through hybridization techniques and PCR technology.

Accordingly, one aspect of the present invention is to provide for nucleic acid hybridization and PCR probes derived from B subtilis Dsb1 which can be used to detect other gram positive microorganism polynucleotide sequences, including genomic and cDNA sequences.

### Example I

### Preparation of a Genomic library

The following example illustrates the preparation of a *Bacillus* genomic library.

Genomic DNA from *Bacillus* cells is prepared as taught in Current Protocols In Molecular Biology vol. 1, edited by Ausubel et al., John Wiley & Sons, Inc. 1987, chapter 2. 4.1. Generally, *Bacillus* cells from a saturated liquid culture are lysed and the proteins removed by digestion with proteinase K. Cell wall debris, polysaccharides, and remaining proteins are removed by selective precipitation with CTAB, and high molecular weight genomic DNA is recovered from the resulting supematant by isopropanol precipitation. If exceptionally clean genomic DNA is desired, an additional step of purifying the *Bacillus* genomic DNA on a cesium chloride gradient is added.

After obtaining purified genomic DNA, the DNA is subjected to Sau3A digestion. Sau3A recognizes the 4 base pair site GATC and generates fragments compatible with several convenient phage lambda and cosmid vectors. The DNA is subjected to partial digestion to increase the chance of obtaining random fragments.

The partially digested *Bacillus* genomic DNA is subjected to size fractionation on a 1% agarose gel prior to cloning into a vector. Alternatively, size fractionation on a sucrose gradient can be used. The genomic DNA obtained from the size fractionation step is purified away from the agarose and ligated into a cloning vector appropriate for use in a host cell and transformed into the host cell.

### Example II

### Detection of gram-positive microorganism disulfide bond isomerase

The following example describes the detection of gram-positive microorganism Dsb1.

DNA derived from a gram-positive microorganism is prepared according to the methods disclosed in Current Protocols in Molecular Biology, Chap. 2 or 3. The nucleic acid is subjected to hybridization and/or PCR amplification with a probe or primer derived from Dsb1.

The nucleic acid probe is labeled by combining 50 pmol of the nucleic acid and 250 mCi of [gamma ³²P] adenosine triphosphate (Amersham, Chicago IL) and T₄ polynucleotide kinase (DuPont NEN®, Boston MA). The labeled probe is purified with Sephadex G-25 super fine resin column (Pharmacia). A portion containing 10⁷ counts per minute of each is used in a typical membrane based hybridization analysis of nucleic acid sample of either genomic or cDNA origin.

The DNA sample which has been subjected to restriction endonuclease digestion is fractionated on a 0.7 percent agarose gel and transferred to nylon membranes (Nytran Plus, Schleicher & Schuell. Durham NH). Hybridization is carried out for 16 hours at 40 degrees C. To remove nonspecific signals, blots are sequentially washed at room temperature under increasingly stringent conditions up to 0.1 x saline sodium citrate and 0.5% sodium dodecyl sulfate. The blots are exposed to film for several hours, the film developed and hybridization patterns are compared visually to detect polynucleotide homologs of *B.subtilis* Dsb1. The homologs are subjected to confirmatory nucleic acid sequencing. Methods for nucleic acid sequencing are well known in the art. Conventional enzymatic methods employ DNA polymerase Klenow fragment, SEQUENASE® (US Biochemical Corp. Cleveland, OH) or Taq polymerase to extend DNA chains from an oligonucleotide primer annealed to the DNA template of interest.

## Claims

1. An expression vector comprising nucleic acid encoding a gram-positive microorganism disulfide isomerase having at least 80% identity to SEQ ID NO: 2, wherein said nucleic acid is under the control of expression signals capable of overexpressing said disulfide isomerase in a gram-positive microorganism.

2. The expression vector of Claim 1, wherein said disulfide isomerase has at least 90% identity to SEQ ID NO: 2.

3. The expression vector of Claim 2, wherein said disulfide isomerase has at least 95% identity to SEQ ID NO: 2.

4. The expression vector of claim 3, wherein said disulfide isomerase has the sequence of SEQ ID NO: 2.

5. The expression vector of Claim 4, wherein said nucleic acid has the sequence of SEQ ID NO: 1.

6. The expression vector of Claim 1 wherein said gram-positive microorganism is a *Bacillus.*

7. The expression vector of Claim 6, wherein the *Bacillus* is selected from the group consisting of *B. subtilis, B. licheniformis, B. lentus, B. brevis, B.* *stearothermophilus, B. alkalophilus, B. amyloliquefaciens, B. coagulans, B. circulans, B. lautus* and *Bacillus thuringiensis.*

8. A gram positive host cell comprising the expression vector of Claim 1.

9. The host cell of Claim 8 that is a *Bacillus.*

10. The host cell of Claim 9 wherein the *Bacillus* is selected from the group consisting of *B. subtilis, B. licheniformis, B. lentus, B. brevis, B. stearothermophilus, B. alkalophilus, B. amyloliquefaciens, B. coagulans, B. circulans, B. lautus* and *Bacillus thuringiensis.*

11. The host cell of Claim 9 wherein said host cell is capable of expressing a heterologous protein.

12. The host cell of Claim 11 wherein said heterologous protein is selected from the group consisting of hormone, enzyme, growth factor and cytokine.

13. The host cell of Claim 12 wherein said heterologous protein is an enzyme.

14. The host cell of Claim 13 wherein said enzyme is selected from the group consisting of proteases, carbohydrases, reductase and lipases; isomerases, transferases, kinases and phophatases.

15. A method for secreting a protein in a gram-positive microorganism comprising the steps of obtaining a gram-positive microorganism host cell comprising an expression vector according to any one of claims 1 to 5, said microorganism further comprising nucleic acid encoding said protein; and culturing said microorganism under conditions suitable for expression of said disulfide bond isomerase and expression and secretion of said protein.

16. The method of Claim 15 wherein said protein is homologous to said host cell.

17. The method of Claim 15 wherein said protein is heterologous to said host cell.

18. The method of Claim 15 wherein said gram-positive organism is a *Bacillus.*

19. The method of Claim 18 wherein said *Bacillus* is selected from the group consisting of *B. subtilis, B. licheniformis, B. lentus, B. brevis, B. stearothermophilus, B. alkalophilus, B. amyloliquefaciens, B. coagulans, B. circulans, B. lautus* and *Bacillus thuringiensis.*

20. The method of Claim 15 wherein said heterologous protein is selected from the group consisting of hormone, enzyme, growth factor and cytokine.

21. The method of Claim 20 wherein said heterologous protein is an enzyme.

22. The method of Claim 21 wherein said enzyme is selected from the group consisting of proteases, carbohydrases, reductase and lipases; isomerases, transferases, kinases and phophatases.

## Patentansprüche

1. Expressionsvektor, umfassend eine Nukleinsäure, die für eine grampositive Mikroorganismus-Disulfidisomerase kodiert, die mindestens 80 % Identität mit SEQ ID NO:2 aufweist, wobei die Nukleinsäure unter der Kontrolle von Expressionssignalen steht, die in der Lage sind, die Disulfidisomerase in einem grampositiven Mikroorganismus zu überexprimieren.

2. Expressionsvektor nach Anspruch 1, wobei die Disulfidisomerase mindestens 90 % Identität mit SEQ ID NO:2 aufweist.

3. Expressionsvektor nach Anspruch 2, wobei die Disulfidisomerase mindestens 95 % Identität mit SEQ ID NO:2 aufweist.

4. Expressionsvektor nach Anspruch 3, wobei die Disulfidisomerase die Sequenz von SEQ ID NO:2 aufweist.

5. Expressionsvektor nach Anspruch 4, wobei die Nukleinsäure die Sequenz von SEQ ID NO:1 aufweist.

6. Expressionsvektor nach Anspruch 1, wobei der grampositive Mikroorganismus ein Bacillus ist.

7. Expressionsvektor nach Anspruch 6, wobei das Bacillus aus der Gruppe bestehend aus B. subtilis, B licheniformis, B. lentus, B. brevis, B. stearothermophilus, B. alkalophilus, B. amyloliquefaciens, B. coagulans, B. circulans, B. lautus und Bacillus thuringiensis ausgewählt ist.

8. Grampositive Wirtszelle, umfassend den Expressionsvektor nach Anspruch 1.

9. Wirtszelle nach Anspruch 8, die ein Bacillus ist.

10. Wirtszelle nach Anspruch 9, wobei der Bacillus aus der Gruppe bestehend aus B. subtilis, B. licheniformis, B. lentus, B. brevis, B. stearothermophilus, B. alkalophilus, B. amyloliquefaciens, B. coagulans, B. circulans, B. lautus und Bacillus thuringiensis ausgewählt ist.

11. Wirtszelle nach Anspruch 9, wobei die Wirtszelle in der Lage ist, ein heterologes Protein zu exprimieren.

12. Wirtszelle nach Anspruch 11, wobei das heterologe Protein aus der Gruppe bestehend aus Hormon, Enzym, Wachstumsfaktor und Cytokin ausgewählt ist.

13. Wirtszelle nach Anspruch 12, wobei das heterologe Protein ein Enzym ist.

14. Wirtszelle nach Anspruch 13, wobei d as Enzym aus der Gruppe bestehend aus Proteasen, Carbohydrasen, Reduktase und Lipasen; Isomerasen, Transferasen, Kinasen und Phosphatasen ausgewählt ist.

15. Verfahren zur Sekretion eines Proteins in einem grampositiven Mikroorganismus, umfassend die Schritte des Erhaltens einer grampositiven Mikroorganismus-Wirtszelle, die einen Expressionsvektor nach einem der Ansprüche 1 bis 5 umfasst, wobei der Mikroorganismus weiter eine Nukleinsäure umfasst, die für das Protein kodiert; und des Züchtens des Mikroorganismus unter Bedingungen, die für die Expression der Disulfidbindungsisomerase und die Expression und Sekretion des Proteins geeignet sind.

16. Verfahren nach Anspruch 15, wobei das Protein homolog zur Wirtszelle ist.

17. Verfahren nach Anspruch 15, wobei das Protein heterolog zur Wirtszelle ist.

18. Verfahren nach Anspruch 15, wobei der grampositive Organismus ein Bacillus ist.

19. Verfahren nach Anspruch 18, wobei das Bacillus aus der Gruppe bestehend aus B. subtilis, B. licheniformis, B. lentus, B. brevis, B. stearothermophilus, B. alkalophilus, B. amyloliquefaciens, B. coagulans, B. circulans, B. lautus und Bacillus thuringiensis ausgewählt ist.

20. Verfahren nach Anspruch 15, wobei das heterologe Protein aus der Gruppe bestehend aus Hormon, Enzym, Wachstumsfaktor und Cytokin ausgewählt ist.

21. Verfahren nach Anspruch 20, wobei das heterologe Protein ein Enzym ist.

22. Verfahren nach Anspruch 21, wobei das Enzym aus der Gruppe bestehend aus Proteasen, Carbohydrasen, Reduktase und Lipasen; Isomerasen, Transferasen, Kinasen und Phosphatasen ausgewählt ist.

## Revendications

1. Vecteur d'expression comprenant un acide nucléique codant pour une isomérase à pont disulfure de micro-organisme Gram positif ayant une identité d'au moins 80 % avec la séquence de NO ID SEQ : 2, dans lequel ledit acide nucléique est sous le contrôle de signaux d'expression capables de surexprimer ladite isomérase à pont disulfure dans un micro-organisme Gram positif.

2. Vecteur d'expression de la Revendication 1, dans lequel ladite isomérase à pont disulfure présente une identité d'au moins 90 % avec la séquence de NO ID SEQ : 2.

3. Vecteur d'expression de la Revendication 2, dans lequel ladite isomérase à pont disulfure présente une identité d'au moins 95 % avec la séquence de NO ID SEQ : 2.

4. Vecteur d'expression de la Revendication 3, dans lequel ladite isomérase à pont disulfure présente la séquence de NO ID SEQ : 2.

5. Vecteur d'expression de la Revendication 4, dans lequel ledit acide nucléique présente la séquence de NO ID SEQ : 1.

6. Vecteur d'expression de la Revendication 1, dans lequel ledit micro-organisme Gram positif est un *Bacillus.*

7. Vecteur d'expression de la Revendication 66, dans lequel le *Bacillus* est sélectionné dans le groupe consistant en *B. subtilis, B. licheniformis, B. lentus, B. brevis, B. stearothermophilus, B. alkalophilus, B.* *amyloliquefaciens, B. coagulans, B. circulans, B. lautus,* et *B. thuringiensis.*

8. Cellule hôte Gram positif comprenant le vecteur d'expression de la Revendication 1.

9. Cellule hôte de la Revendication 8 qui est un *Bacillus*.

10. Cellule hôte de la Revendication 9 dans lequel le *Bacillus* est sélectionné dans le groupe consistant en *B. subtilis, B. licheniformis, B. lentus, B. brevis, B. stearothermophilus, B. alkalophilus, B. amyloliquefaciens, B. coagulans, B. circulans, B. lautus,* et *B. thuringiensis.*

11. Cellule hôte de la Revendication 9 dans lequel ladite cellule hôte est capable d'exprimer une protéine hétérologue.

12. Cellule hôte de la Revendication 11 dans lequel ladite protéine hétérologue est sélectionnée dans le groupe consistant en hormone, enzyme, facteur de croissance et cytokine.

13. Cellule hôte de la Revendication 12 dans lequel ladite protéine hétérologue est une enzyme.

14. Cellule hôte de la Revendication 13 dans lequel ladite enzyme est sélectionnée dans le groupe consistant en protéases, carbohydrases, réductase et lipases ; isomérases, transférases, kinases et phosphatases.

15. Procédé pour sécréter une protéine dans un micro-organisme Gram positif comprenant les étapes d'obtention d'une cellulose hôte de micro-organisme Gram positif comprenant un vecteur d'expression selon l'une quelconque des revendications 1 à 5, ledit micro-organisme comprenant en outre un acide nucléique codant pour ladite protéine ; et de mise en culture dudit micro-organisme dans des conditions adaptées pour l'expression de ladite isomérase à pont disulfure et l'expression et la sécrétion de ladite protéine.

16. Procédé de la Revendication 15 dans lequel ladite protéine est homologue à ladite cellule hôte.

17. Procédé de la Revendication 15 dans lequel ladite protéine est hétérologue à ladite cellule hôte.

18. Procédé de la Revendication 15 dans lequel ledit organisme Gram positif est un *Bacillus.*

19. Procédé de la Revendication 18 dans lequel ledit *Bacillus* est sélectionné dans le groupe consistant en *B. subtilis, B. licheniformis, B. lentus, B. brevis, B. scearothermophilus, B. alkalophilus, B. amyloliquefaciens, B. coagulans, B. circulans, B. lautus*, et *B. thuringiensis*.

20. Procédé de la Revendication 15 dans lequel ladite protéine hétérologue est sélectionnée dans le groupe consistant en hormone, enzyme, facteur de croissance et cytokine.

21. Procédé de la Revendication 20 dans lequel ladite protéine hétérologue est une enzyme.

22. Procédé de la Revendication 21 dans lequel ladite enzyme est sélectionnée dans le groupe consistant en protéases, carbohydrases, réductase et lipases ; isomérases, transférases, kinases et phosphatases.
